# FASCICULE DE BREVET EUROPEEN

(11) **EP 1 313 500 B1**
(45) Date de publication et mention de la délivrance du brevet: **13.10.2004**
(21) Numéro de dépôt: 01963045.8
(22) Date de dépôt: 02.08.2001
(51) Int. Cl.: A61K 38/04, A61P 31/04

(54) **ASSOCIATIONS DALFOPRISTINE/QUINUPRISTINE AVEC LE CEFPIROME**
ZUSAMMENSETZUNGEN AUS DALFOPRISTIN UND QUINUPRISTIN MIT CEFPIROM
COMBINATIONS OF DALFOPRISTINE/QUINUPRISTINE WITH CEFPIROME

(30) Priorité: 03.08.2000 FR 0010245
(43) Date de publication de la demande: 28.05.2003
(73) Titulaire: King Pharmaceuticals, Inc., Bristol, TN 37620 (US)
(72) Inventeur: FEGER, Céline, F-75013 Paris (FR); MOREILLON, Philippe, CH-1000 Lausanne 26 (CH); VOUILLAMOZ, Jacques, CH-1096 Cully (CH)
(74) Mandataire: Rousseau, Pierrick Edouard
(86) Numéro de dépôt international: PCT/FR2001/002520
(87) Numéro de publication internationale: WO 2002/011751

(56) Documents cités:
- VOUILLAMOZ, J. ET AL: "Quinupristin - dalfopristin combined with beta-lactams for treatment of experimental endocarditis due to Staphylococcu aureus constitutively resistant to macrolide- lincosamide -streptogramin B antibiotics." ANTIMICROBIAL AGENTS AND CHEMOTHERAPY, (JULY, 2000) VOL. 44, NO. 7, PP. 1789-1795. PRINT., XP001001441

## Description

La présente invention concerne des associations de quinupristine et dalfopristine avec le cefpirome, qui manifestent une synergie d'action aussi bien bactériostatique que bactéricide.

La présente invention concerne aussi des compositions pharmaceutiques injectables pour l'administration parentérale de quinupristine et dalfopristine, combinée avec le cefpirome, ainsi que des kits de présentation des principes actifs objets de l'association selon l'invention.

Le brevet européen EP 248,703 décrit des dérivés de streptogramines du groupe B de formule générale : 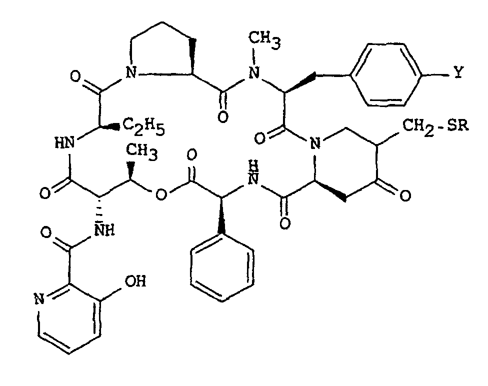 ainsi que leurs combinaisons avec des dérivés de streptogramines du groupe A de structure : 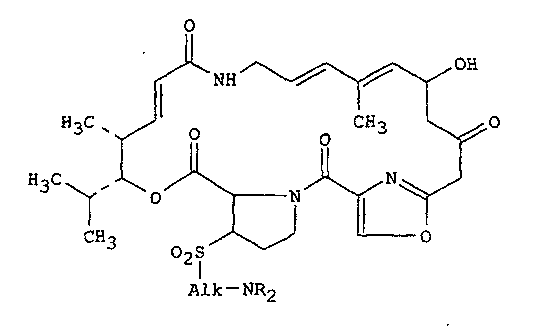 Les dérivés de streptogramines du groupe A sont décrits plus en détail dans le brevet européen EP 191,662.

La quinupristine, dérivé de pristinamycine I (streptogramine du groupe B), et la dalfopristine, dérivé. de pristinamycine II (streptogramine du groupe A), sont les composantes du Synercid® :

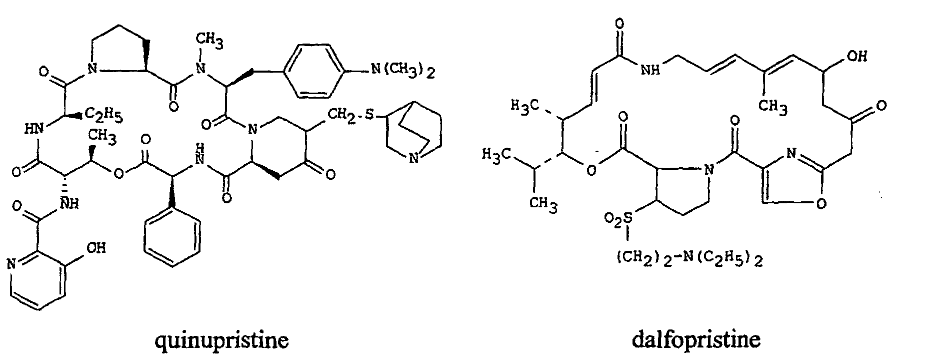

Le Synercid® (quinupristine / dalfopristine) est une combinaison 30/70, injectable, efficace sur la plupart des germes à gram-positif, en particulier *Staphylococcus aureus* résistants à la méticilline (MRSA), *Staphylococcus aureus* Macrolide-Lincosamide-Streptogramine B sensibles (MLS_{B}) et *Enterococcus faecium* résistants à la vancomycine (VREF). Son activité antibactérienne, notamment sur les germes résistants à la vancomycine, est citée dans de nombreuses publications [The Annals of Pharmacotherapy, 29, 1022-1026 (1995) ; Microbial Drug resistance, 1, 223-234 (1995) ; Antimicrobial Agents Chemother., 39, 1419-1424 ( 1995) ...]. Cependant, le Synercid perd son activité bactéricide sur les staphylocoques constitutivement résistants aux antibiotiques du groupe Macrolide-Lincosamide-Streptogramine B (C-MLS_{B} résistants).

La demande internationale WO 98/22107 décrit la préparation de compositions pharmaceutiques stabilisées comprenant la combinaison quinupristine/dalfopristine, sous forme de sel obtenu par addition d'acide methanesulphonique ou chlorhydrique dans des quantités au moins stoechiométriques et à un pH compris dans l'intervalle [3,5 ; 5].

Dans l'environnement clinique, certaines bactéries (le MRSA C-MLS_{B} résistant par exemple) peuvent compromettre l'efficacité de la combinaison quinupristine/dalfopristine si des concentrations adéquates de dalfopristine ne sont pas présentes sur le site de l'infection. L'un des moyens pour résoudre le problème a été l'utilisation d'un nombre croissant de doses de quinupristine/dalfopristine dans une periode de 24 heures ou d'utiliser un système de perfusion continue.

Le cefpirome : sulfate de l'acide (Z)-7-[2-(2-aminothiazol-4-yl)-2-méthoxyimino-acétamido]-3-(1-pyridiniométhyl)-3-cephem-4-carboxylique [Martindale, 32^{nd} édition, Kathleen Parfitt Ed.] est un β-lactame, de la classe des cephalosporines de 4^{è} génération, doué d'une bonne activité sur les *Staphylococcus aureus* méticilline resistants (MRSA) par comparaison aux autres produits de cette classe. Il est habituellement administré par voie parentérale par injection i.v. en 3 à 5 minutes ou par perfusion sur une durée de 20 à 30 minutes, à raison de doses de cefpirome-sulfate de 1 à 2 g par 12 heures en fonction de la sévérité de l'infection. II est particulièrement indiqué dans le traitement des infections du tractus urinaire, du tractus respiratoire, des infections de la peau et aussi dans le traitement des septicémies et des infections des immunodéprimés.

Il a été maintenant trouvé, ce qui fait l'objet de la présente invention, que les combinaisons de quinupristine/dalfopristine avec le cefpirome peuvent avoir un intérêt particulier dans le traitement d'infections très difficiles à traiter ou mettant en jeu la vie du patient, et qui demandent une activité bactéricide rapide et efficace, comme ce peut être le cas de combinaisons manifestant une synergie d'action contre les bactéries.

La synergie apporte une puissance beaucoup plus élevée et peut, par exemple, permettre de diminuer la concentration en quinupristine/dalfopristine ou en cefpirome, ou d'augmenter l'intervalle d'administration des doses nécessaires à inhiber ou éradiquer ladite bactérie (plus particulièrement vis à vis des staphylocoques multi résistants comme notamment les souches résistantes à la méticilline). La synergie peut également permettre de traiter des infections sur lesquelles chacun des principes actifs administrés en monothérapie n'aurait pas eu d'efficacité.

Le Synercid® (quinupristine/dalfopristine - ci-après Q-D) et le cefpirome ont été testés séparément chez le rat sur des endocardites expérimentales dues à des MRSA C-MLS_{B} résistants.
Sur les microorganismes étudiés, aucun des produits n'a manifesté d'efficacité in vivo en monothérapie. En revanche, l'association quinupristine/dalfopristine avec le cefpirome est efficace sur plus de 90% des rats traités.

L'association quinupristine/dalfopristine avec le cefpirome est particulièrement intéressante du fait qu'elle est appelée à devenir aussi un traitement de choix pour les patients nécessitant un traitement simultané sur les germes à gram positif et à gram négatif.

### DÉTERMINATION EXPERIMENTALE

### Matériel et méthodes

Les études mises en oeuvre in vitro, et in vivo chez l'animal, sont décrites ci-après.

Les microorganismes testés sont 2 isolats cliniques de MRSA C-MLS_{B} résistants : souches AW7 et P8, préalablement caractérisées in vitro, et in vivo dans l'endocardite expérimentale du rat.

L'étude in vitro et la détermination des CMIs (concentrations minimales inhibitrices) est effectuée à partir de cultures sur plaques d'agar [Antimicrob. Agents Chemother., 39, 1419-1424 (1995)].

Pour l'étude de l'endocardite expérimentale, des rats femelles Wistar de 200 g avec des végétations aortiques [produites selon la méthode décrite par Heraief et al., Infect Immunol., 37, 127-31 (1982)] ont été infectés avec un inoculum de 10⁵ CFU/ml de l'organisme à tester correspondant à 10 fois l'inoculum minimum pour produire une endocardite chez au moins 90% des contrôles non traités.
Le traitement est débuté 12 heures après l'infection bactérienne ; il est poursuivi pendant 5 jours. Les antibiotiques sont administrés au moyen d'un cathéter veineux permanent, avec une pompe programmable (pompe 44 ; Harvard Apparatus Inc.) selon la méthode décrite dans Antimicrobial Agents Chemother., 39, 1419-1424 (1995). Les traitements effectués simulent une cinétique chez l'homme, produite soit par administration intraveineuse de 7 mg/kg de quinupristine/dalfopristine toutes les 12 heures ou de 2 g de cefpirome par voie i.v. toutes les 12 heures. Le dosage relativement peu élevé de quinupristine/dalfopristine a été choisi de manière à mettre en évidence, le cas échéant, une synergie avec le cefpirome.
Préalablement aux expériences de traitement, la mise au point de la simulation de la cinétique humaine de l'association quinupristine/dalfopristine avec cefpirome, chez le rat, s'effectue sur des animaux cathétérisés mais non infectés. Dans ces expériences préliminaires, chaque point de la courbe de phannacocinétique est la moyenne (± déviation standard) des concentrations mesurées chez 6 rats différents. Les concentrations en produit dans le sérum, sont mesurées dans un bioessai (utilisation de *Micrococcus luteus* pour quinupristine/dalfopristine et de *Bacillus subtilis* ATCC 6633 pour cefpirome).

Les concentrations en quinupristine/dalfopristine dans le sérum des rats sont de 5 mg/l, 1 heure après le début du traitement et décroissent progressivement jusqu'à des concentrations indétectables après 6 heures. Les concentrations en cefpirome sont de 160,6 ± 19,7 mg/l, 5 minutes après le début du traitement et décroissent progressivement jusqu'à 2,2 ±1,4 mg/l après 12 heures. Ces valeurs sont conformes aux niveaux thérapeutiques observés chez l'homme pour ces principes actifs. De plus durant chaque expérience de traitement ces concentrations sont contrôlées chez 3 rats pris au hasard pour chaque temps de référence de la pharmacocinétique.

Les rats contrôles sont sacrifiés au début du traitement et les rats traités sont sacrifiés 24 heures après la dernière administration d'antibiotique. La densité bactérienne dans les végétations est déterminée après dissection des végétations dans des conditions stériles, pesée, homogénéisation dans 1 ml de solution saline, puis dilution de la solution (série de dilutions) avant d'être déposée sur des plaques pour comptage des colonies. Le nombre de colonies croissant sur les plaques est déterminé après 48 heures d'incubation à 35°C. Les densités bactériennes dans les végétations sont exprimées en log₁₀ CFU/g de tissu. Le niveau de détection minimum est ≥ 2 log₁₀ CFU/g de végétation. L'éventualité d'une émergence de résistance est également vérifiée. Les densités bactériennes moyennes dans les végétations des différents groupes sont comparées par la méthode de Kruskal-Wallis complétée par la méthode de Dunn. Les différences ont été considérées comme significatives lorsque P est ≤ 0,05.

### Résultats

Les concentrations minimum inhibitrices (CMIs) de quinupristine/dalfopristine sur les souches AW7 et P8 de MRSA sont 0,5 mg/l pour chacune des souches et les CMIs pour le cefpirome sont respectivement de 4 mg/l sur AW7 et 2 mg/l sur P8.

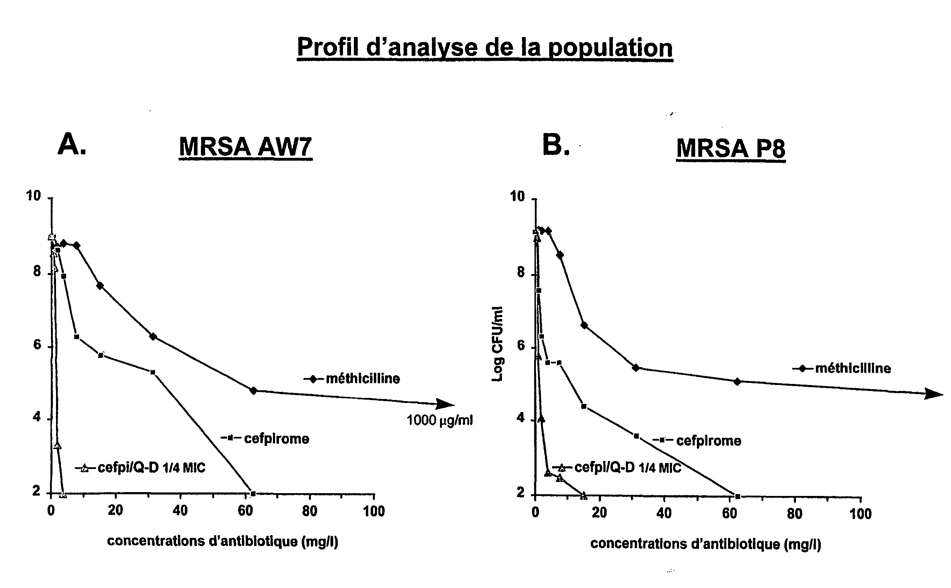

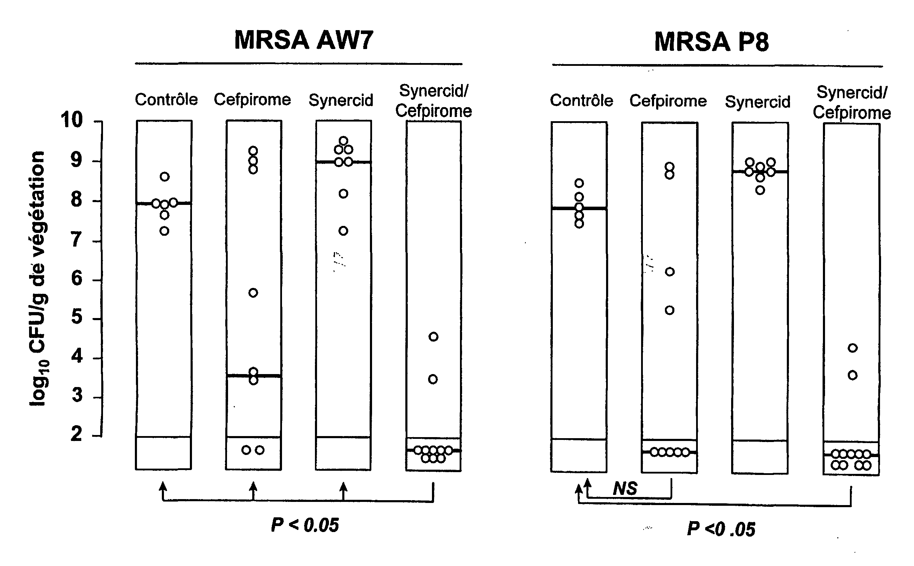

Des populations bactériennes des souches AW7 et P8 de MRSA peuvent croître sur des plaques contenant jusqu'à 60 mg/l de cefpirome. On observe, après addition aux plaques de concentrations subinhibitrices (¼CMI - concentration non efficace) de la ne peut pas croître sur des plaques contenant des concentrations ≥ 15 mg/l de cefpirome.
La figure I, ci-après jointe en annexe, illustre cette synergie d'action.

L'étude in vivo dans l'endocardite expérimentale du rat a permis également de mettre en évidence une synergie.
La figure II ci-après jointe en annexe, montre que ni quinupristine/dalfopristine, ni le cefpirome en monothérapie ne sont significativement efficaces sur les endocardites aortiques dues aux souches AW7 et P8 de MRSA (P>0,05) ; en revanche l'association de quinupristine/dalfopristine avec cefpirome s'est montrée significativement efficace (P<0,05) sur chacune d'entre elles (log₁₀ CFU/g de végétation ≤ 2 dans plus de 80% des cas) comme le montrent également les tableaux ci-dessous.

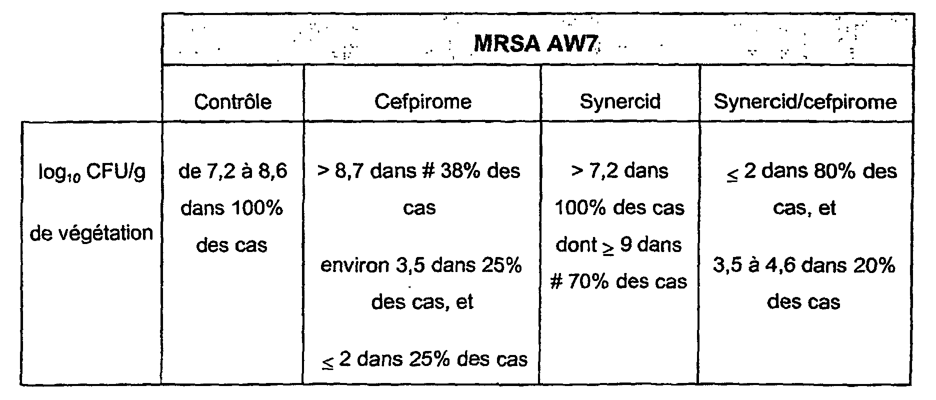

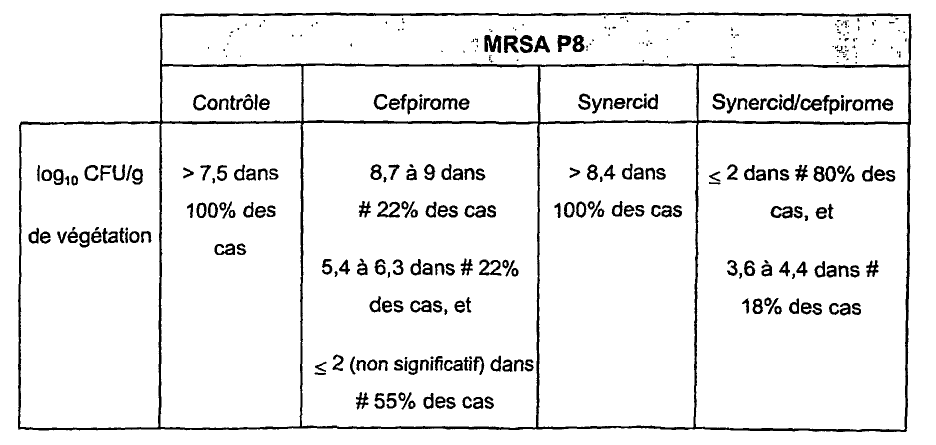

De ce fait le traitement par une telle association permet d'une part d'atteindre une efficacité qui n'était pas obtenue en monothérapie et/ou, d'autre part, permet l'utilisation de doses plus faibles des produits associés ou le choix d'intervalles plus larges entre les administrations, avec une meilleure efficacité (bactériostatique et bactéricide) que celle qui était atteinte en monothérapie. Notamment dans le cas d'infections sévères (comme par exemple les endocardites), la synergie permet d'utiliser le cefpirome à des doses usuelles, alors qu'en l'absence de l'association synergisante, il aurait été nécessaire, pour obtenir un efficacité, d'augmenter les doses à un niveau tel que le cefpirome n'aurait pas pu être utilisé.

Les doses de principe actif (Synercid® : combinaison quinupristine + dalfopristine) administré aux patients sont comprises entre 5 et 15 mg/kg toutes les 12 heures, mais sont en général et de préférence 5 à 7,5 mg/kg toutes les 8 heures.

La dose de cefpirome habituellement administrée aux patients est de 2 à 4 g par jour en 2 perfusions ou en 2 injections intraveineuses directes, soit 1 à 2 g toutes les 12 heures en fonction de la sévérité de l'infection.

Dans les associations ci-dessus les combinaisons quinupristine/dalfopristine avec le cefpirome peut être avantageusement utilisées, pour le traitement des patients, en injection continue ou à raison de plusieurs administrations. Les doses choisies dépendent de l'infection bactérienne à traiter et de l'effet de synergie recherché.
Lorsque la synergie permet, grâce à l'association, d'obtenir un effet sur des germes qui n'auraient pas pu être traités en monothérapie par l'un ou l'autre des principes actifs, les doses associées peuvent être choisies dans l'intervalle de doses habituellement utilisées en monothérapie ou le cas échéant des doses inférieures selon la nature l'infection. A savoir des doses journalières de quinupristine/dalfopristine dans l'intervalle 15 à 30 ou 60 mg/kg ou dans l'intervalle 10 à 30 ou 60 mg/kg en doses fractionnées ou en injection continue et des doses journalières de cefpirome de 2 à 4 g en 2 perfusions ou en 2 injections intraveineuses directes.
Lorsque la synergie permet, grâce à l'association, de traiter des infections bactériennes à des doses moins élevées de quinupristine/dalfopristine et/ou de cefpirome, donc avec moins de risques d'effets secondaires (par exemple dans le cas de germes présentant moins de résistance), les doses associées de quinupristine/dalfopristine et de cefpirome peuvent être choisies dans un intervalle réduit vis à vis de la dose maximum utilisable en monothérapie. A savoir des doses de quinupristine/dalfopristine comprises entre 10 et 22,5 à 30 mg/kg maximum en doses fractionnées ou en injection continue et des doses de cefpirome comprises entre 2 et 4g en 2 perfusions ou en 2 injections intraveineuses directes.

Il est entendu que les associations peuvent inclure plusieurs types d'administration tels que la coadministration simultanée, l'administration successive à des temps différés ou l'administration via des cathéters multi-lumière (multicanaux).

Pour le traitement, les formulations de quinupristine/dalfopristine sont présentées sous forme liquide, lyophilisées ou congelées.
Les compositions lyophilisées peuvent être reprises au moment de l'emploi, dans l'eau pour préparation injectable (eau ppi) ou dans tout autre milieu injectable compatible, en particulier en milieu glucosé (solution aqueuse de glucose à 5% par exemple), ou à titre non limitatif, dans des solutions de dextran, des solutions de polyvinylpyrrolidone, ou des solutions de polysorbate 80. Selon un mode préféré de l'invention, les formulations sont reprises en solution par passage intermédiairement par une solution concentrée (de 50 à 250 mg/ml, de préference environ 100 mg/ml) ci-après appelée "concentrat" ; cette solution est diluée au moment de l'emploi dans un milieu pour injection tel que décrit ci-dessus pour une administration sous forme de perfusion ; il est également possible de reprendre le lyophilisat dans de l'eau ppi, puis de diluer le concentrat ainsi obtenu dans le milieu d'injection choisi.

Les compositions pharmaceutiques peuvent être congelées à partir des solutions initialement préparées (5 à 250 mg/ml) ou à partir de solutions préalablement diluées (pour la préparation de poches congelées par exemple). Elles sont décongelées au moment de l'utilisation puis diluées, si nécessaire. Les solutions présentées à l'état liquide contiennent 5 à 250 mg/ml de principe actif. Elles sont diluées au moment de l'emploi, jusqu'à des concentrations comprises entre 0,5 et 10 mg/ml.

Selon l'invention, les formulations de quinupristine/dalfopristine, éventuellement sous forme de solution concentrée, ou sous forme de solution diluée peuvent être associées pour la coadministration avec une solution de cefpirome au moment de l'injection. L'association peut être faite sous forme de 2 poches de perfusion, l'une contenant la quinupristine/dalfopristine dans son milieu d'injection et l'autre contenant la solution de cefpirome, ou par utilisation de 2 seringues l'une contenant quinupristine/dalfopristine et l'autre contenant la solution de cefpirome, ou encore alternativement, l'association peut être faite par utilisation de l'un des principes actifs dans une poche de perfusion et l'autre dans une seringue.

Il est entendu que la présentation en kits pour la formulation de quinupristine/dalfopristine et de cefpirome entre aussi dans le cadre de la présente invention.

Toute forme de kits de présentation peut convenir, notamment, à titre d'exemple des présentations sous forme d'un double flaconnage, des présentations sous forme de poches de perfusion contenant les principes actifs, des présentations sous forme d'une poche de perfusion contenant l'un des principe actifs et d'un flacon ou d'une ampoule contenant l'autre principe actif, d'un ou plusieurs flacons comprenant l'un des principe actifs (par exemple comprenant le lyophilisat de quinupristine/dalfopristine) et un flacon ou une ampoule contenant l'autre principe actif. Des dispositifs du type seringue à double compartiment, peuvent aussi s'avérer particulièrement adaptés.

## Revendications

1. Association ayant une synergie d'action **caractérisée en ce qu'**elle est constituée d'une combinaison de quinupristine/dalfopristine avec le cefpirome.

2. Compositions pharmaceutiques injectables pour l'administration parentérale de quinupristine et dalfopristine, combinée avec le cefpirome.

3. Compositions pharmaceutiques injectables selon la revendication 2, **caractérisée en ce qu'**elles sont destinées à une administration intraveineuse.

4. Associations selon la revendication 1, **caractérisées en ce que** quinupristine/dalfopristine et cefpirome sont administrés simultanément,ou successivement.

5. Kit de présentation de l'association quinupristine/dalfopristine avec le cefpirome.

6. Kit de présentation selon la revendication 5, **caractérisé en ce qu'**il est destiné à une administration parentérale.

7. Kit de présentation selon la revendication 6, **caractérisé en ce qu'**il s'agit d'un double flaconnage, de poches de perfusion contenant les principes actifs, d'une poche de perfusion contenant l'un des principe actifs et d'un flacon ou d'une ampoule contenant l'autre principe actif, d'un ou plusieurs flacons comprenant l'un des principe actifs et un flacon ou une ampoule contenant l'autre ou encore d'une seringue à double compartiment

## Patentansprüche

1. Assoziation mit synergetischer Wirkung, **dadurch gekennzeichnet, dass** sie von einer Kombination aus Quinupristine/Dalfopristine und Cefpirome gebildet wird.

2. Pharmazeutische Zusammensetzungen, die zur parenteralen Verabreichung von Quinupristine und Dalfopristine in Kombination mit Cefpirome injiziert werden können.

3. Pharmazeutische injizierbare Zusammensetzungen nach Anspruch 2, **dadurch gekennzeichnet, dass** sie für eine intravenöse Verabreichung bestimmt sind.

4. Assoziationen nach Anspruch 1, **dadurch gekennzeichnet, dass** Quinupristine/Dalfopristine und Cefpirome gleichzeitig oder nacheinander verabreicht werden.

5. Darreichungskit für die Assoziation Quinupristine/Dalfopristine und Cefpirome.

6. Darreichungskit nach Anspruch 5, **dadurch gekennzeichnet, dass** er für eine parenterale Verabreichung bestimmt ist.

7. Darreichungskit nach Anspruch 6, **dadurch gekennzeichnet, dass** er aus einem der Folgenden besteht: einem Doppelbehälter; die Wirkstoffe enthaltenden Infusionsbeuteln; einem den einen Wirkstoff enthaltenden Infusionsbeutel und einer den anderen Wirkstoff enthaltenden Flasche oder Ampulle; einer oder mehreren den einen Wirkstoff enthaltenden Flasche(n) und einer den anderen Wirkstoff enthaltenden Flasche oder Ampulle; oder aus einer Zweikammerspritze.

## Claims

1. An association having a synergistic action, **characterised in that** it consists of a combination of quinupristin/dalfopristin with cefpirome.

2. Injectable pharmaceutical compositions for the parenteral administration of quinupristin and dalfopristin, combined with cefpirome.

3. Injectable pharmaceutical compositions according to Claim 2, **characterised in that** they are intended for intravenous administration.

4. Associations according to Claim 1, **characterised in that** quinupristin/dalfopristin and cefpirome are administered simultaneously, or in succession.

5. A presentation kit for the association of quinupristin/dalfopristin with cefpirome.

6. A presentation kit according to Claim 5, **characterised in that** it is intended for parenteral administration.

7. A presentation kit according to Claim 6, **characterised in that** it is a double bottle, perfusion bags containing the active ingredients, a perfusion bag containing one of the active ingredients and a vial or an ampoule containing the other active ingredient, one or more vials comprising one of the active ingredients and a vial or an ampoule containing the other or alternatively a dual-compartment syringe.
